# EUROPEAN PATENT APPLICATION

(11) **EP 4 653 420 A1**
(43) Date of publication of application: **26.11.2025**
(21) Application number: 24744311.2
(22) Date of filing: 17.01.2024
(51) Int. Cl.: C07C 269/06, C07C 271/22, C07C 271/10

(54) **METHOD FOR PREPARING CHIRAL AMINO ALCOHOL PHARMACEUTICAL INTERMEDIATES**

(30) Priority: 17.01.2023 CN 202310060397
(71) Applicant: SHENYANG HAISCO PHARMACEUTICAL CO., LTD., Shenyang, Liaoning 110168 (CN)
(72) Inventor: JIANG, Xi, Shenyang, Liaoning 110168 (CN); DOU, Ying, Shenyang, Liaoning 110168 (CN); FAN, Jiang, Shenyang, Liaoning 110168 (CN); HU, Jianyong, Shenyang, Liaoning 110168 (CN); LIN, Hanwen, Shenyang, Liaoning 110168 (CN)
(74) Representative: AWA Denmark A/S
(86) International application number: PCT/CN2024/072810
(87) International publication number: WO 2024/153137

(57) **Abstract**

Provided in the present invention is a method for preparing chiral amino alcohol pharmaceutical intermediates as represented by formula A and formula A2 or salts thereof. Compared with a chiral resolution preparation method in the prior art, the preparation method of the present invention has the advantages of mild reaction conditions, a simple operation, a high reaction yield, high product purity, high chiral purity and convenient post-treatment, and is suitable for industrial production.

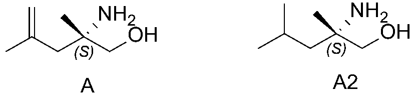

## Description

### Technical Field

The present invention relates to a method for preparing a pharmaceutical intermediate, and in particular to a method for preparing a chiral amino alcohol pharmaceutical intermediate, which belongs to the technical field of pharmaceutical chemistry.

### Background Art

Chiral amino alcohol fragments are widely used as key intermediates or starting materials in the production and preparation of pharmaceuticals. For example, compounds related to the AAK1 target developed by Bristol-Myers Squibb Company all include chiral amino alcohol fragments. In the prior art, chiral amino alcohols are mainly prepared by the route of chiral resolution of racemic amino alcohols (or derivatives).

Route 1: For the saturated chiral amino alcohol compound 35, the literature J. Med. Chem. 2022, 65, 4457-4480. reported that (S)-36 obtained by chiral resolution was subjected to hydrogenation to remove the Cbz protecting group and reduce the double bond to give compound 37, and then subsequent operations such as Boc protection were performed to give the saturated amino alcohol 35 (equivalent to A2). The synthetic route employs chiral resolution, which is unfavourable for cost control and commercial scale-up.

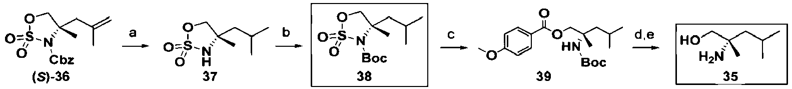

Route 2: No patents or literature have reported on the unsaturated chiral amino alcohol compound 35 to date. The analogous patent WO 2005087731 A1 discloses similar intermediates, but the process adopts a column chromatography approach, and does not disclose a method for obtaining unsaturated chiral amino alcohols. The use of column chromatography for separation and purification is unfavourable for cost control and commercial scale-up.

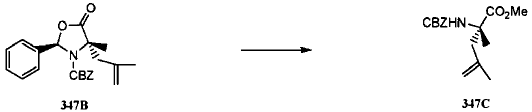

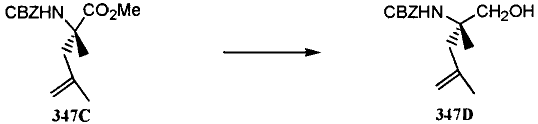

The preparation processes of the above existing routes are unfavourable for cost control and commercial scale-up production. Based on the wide range of application prospects of chiral amino alcohols, the inventors have developed a method for preparing a chiral amino alcohol and an intermediate thereof. The method has the advantages of mild reaction conditions, simple operation, high reaction yield, high product purity, high chiral purity and convenient post-treatment, and is suitable for industrial production.

### Summary of the Invention

The present invention provides a method for preparing an intermediate represented by formula A or a salt thereof, comprising step d1 of subjecting a compound of formula B-1 to a hydrolysis reaction under alkaline conditions to remove the Cbz protecting group to obtain the intermediate of formula A:

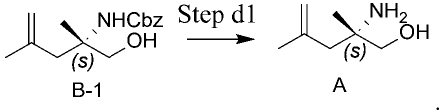

In some embodiments, the hydrolysis reagent for the hydrolysis reaction is selected from one or more of sodium hydroxide, potassium carbonate, potassium tert-butoxide, potassium tert-pentoxide, sodium carbonate, caesium carbonate, caesium hydroxide, lithium hydroxide, potassium hydroxide, sodium methoxide or sodium ethoxide, preferably sodium hydroxide or potassium tert-butoxide, more preferably sodium hydroxide.

In some embodiments, the solvent for the hydrolysis reaction is selected from one or more of a polar aprotic solvent or a non-polar solvent.

In some embodiments, the solvent for the hydrolysis reaction is selected from one or more of N,N-dimethylformamide, N,N-dimethylacetamide, N-methylpyrrolidone, dichloromethane, 1,2-dichloroethane, ethyl acetate, methyl tert-butyl ether, dimethylsulfoxide, acetonitrile, diethyl ether, tetrahydrofuran, ethanol, methanol or water, preferably at least one of ethanol, tetrahydrofuran or water, more preferably at least one of ethanol or water.

In some embodiments, the reaction temperature of the hydrolysis reaction is 0°C-90°C, preferably 50°C-80°C, more preferably 70°C ± 5°C.

The present invention also provides a method for preparing an intermediate represented by formula A2 or a salt thereof, comprising step d2 of subjecting a compound of formula B-1 to a hydrogenation reduction reaction to obtain the intermediate of formula A2:

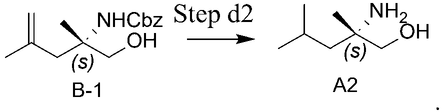

In some embodiments, the reducing agent used in the reduction reaction is 5% or 10% Pd/C.

In some embodiments, the reducing agent used is 10% Pd/C, and the amount thereof is 1%-50%, preferably 10%-30%, by mass of the compound of formula B-1.

In some embodiments, the solvent used in the reduction reaction is selected from one or more of N,N-dimethylformamide, N,N-dimethylacetamide, N-methylpyrrolidone, dichloromethane, 1,2-dichloroethane, ethyl acetate, methyl tert-butyl ether, dimethylsulfoxide, acetonitrile, diethyl ether, tetrahydrofuran, ethanol, methanol or water, preferably one or more of ethanol, methanol, ethyl acetate or dichloromethane, more preferably methanol.

In some embodiments, the reaction temperature of the reduction reaction is 20°C-60°C, preferably 20°C-40°C.

Further, the present invention provides a method for preparing an intermediate of formula B-1, comprising step c of reacting a compound of formula C-1 in the presence of an active agent to obtain a mixed anhydride, followed by reduction to obtain the intermediate of formula B-1:

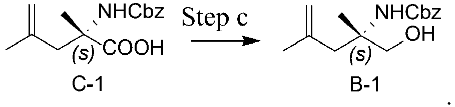

In some embodiments, the active agent is selected from methyl chloroformate, ethyl chloroformate, isopropyl chloroformate, butyl chloroformate, isobutyl chloroformate, tert-butyl chloroformate, N,N'-dicyclohexylcarbodiimide or N,N'-carbonyldiimidazole, preferably methyl chloroformate or isobutyl chloroformate, more preferably isobutyl chloroformate.

In some embodiments, the base used during the preparation of the mixed anhydride is selected from potassium carbonate, sodium carbonate, triethylamine, N,N-diisopropylethylamine or diethylamine, preferably triethylamine.

In some embodiments, the solvent for the reaction in step c is selected from a polar aprotic solvent or a polar protic solvent.

In some embodiments, the solvent for the reaction in step c is selected from one or more of N,N-dimethylformamide, N-methylpyrrolidone, acetonitrile, tetrahydrofuran, 2-methyltetrahydrofuran, acetone, water, methanol or ethanol, preferably tetrahydrofuran.

In some embodiments, the solvent is selected from water or a mixture of water and a polar solvent.

In some embodiments, the polar solvent is selected from tetrahydrofuran, methanol or ethanol.

In some embodiments, the reducing agent used in the reaction of step (c) is selected from sodium borohydride or lithium borohydride, preferably sodium borohydride.

In some embodiments, the additive used in the reaction of step (c) is selected from methanol, ethanol or water, preferably methanol or water, more preferably water.

In some embodiments, the molar ratio of formula B-1 : isobutyl chloroformate : triethylamine : sodium borohydride is 1.0:(1.0-3.0):(1.0-4.0):(1.0-6.0), preferably the molar ratio is 1.0:(1.3-2.5):(1.5-3.0):(2.0-4.0), more preferably the molar ratio is 1.0:(1.3-1.7):(1.5-2.0):(2.5-3.0).

In some embodiments, the temperature of the reaction in step c is 20°C-40°C.

Further, the present invention provides a method for preparing an intermediate of formula C-1, comprising step b of reacting a compound of formula D-1 in the presence of an alkaline reagent to obtain the intermediate of formula C-1: 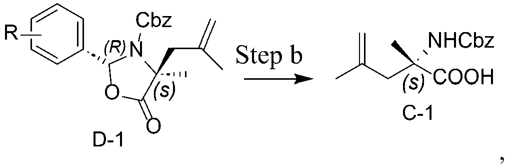 wherein R is selected from H, halogen, C₁₋₄ alkyl, C₁₋₄ alkoxy, C₃₋₆ carbocyclyl, or 4- to 6-membered heterocyclyl, and the alkyl, alkoxy, carbocyclyl or heterocyclyl is optionally substituted with 1 to 4 substituents selected from F, Cl, Br, I, OH, NH₂, C₁₋₄ alkyl, C₁₋₄ alkoxy or C₃₋₆ cycloalkyl.

In some embodiments, the R in formula D-1 is H.

In some embodiments, the alkaline reagent in step b is selected from one or more of sodium hydroxide, potassium carbonate, potassium tert-butoxide, potassium tert-pentoxide, sodium carbonate, caesium carbonate, caesium hydroxide, lithium hydroxide, potassium hydroxide, sodium methoxide or sodium ethoxide, preferably lithium hydroxide.

In some embodiments, the solvent used in the reaction of step b is selected from one or more of amide solvents, alkane solvents, halogenated alkane solvents, alcohol solvents, ketone solvents, ester solvents, ether solvents, nitrile solvents, sulfone solvents or water, preferably one or more of N,Ndimethylformamide, *N,N-*dimethylacetamide, N-methylpyrrolidone, dichloromethane, 1,2-dichloroethane, ethyl acetate, acetone, methanol, ethanol, isopropanol, n-butanol, trifluoroethanol, ethylene glycol, n-butanone, methyl tert-butyl ether, dimethylsulfoxide, acetonitrile, diethyl ether, tetrahydrofuran and water, more preferably at least one of tetrahydrofuran or water.

In some embodiments, the temperature of the reaction in step b is -10°C-50°C, preferably 0°C-30°C.

Still further, the present invention provides a method for preparing a compound of formula D-1, comprising step a of reacting a compound of formula E-1 and a compound of formula F-1 in the presence of an alkaline reagent to obtain the compound of formula D-1: 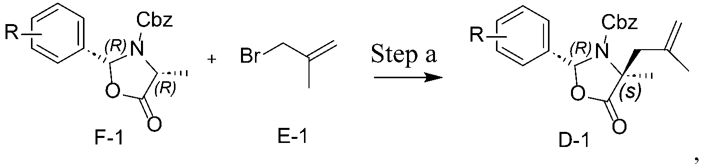 wherein R is selected from H, halogen, C₁₋₄ alkyl, C₁₋₄ alkoxy, C₃₋₆ carbocyclyl, or 4- to 6-membered heterocyclyl, and the alkyl, alkoxy, carbocyclyl or heterocyclyl is optionally substituted with 1 to 4 substituents selected from F, Cl, Br, I, OH, NH₂, C₁₋₄ alkyl, C₁₋₄ alkoxy or C₃₋₆ cycloalkyl.

In some embodiments, the R in formulae F-1 and D-1 is H.

In some embodiments, the alkaline reagent for the reaction in step a is selected from one or more of sodium hydride, lithium diisopropylamide, lithium amide, lithium bis(trimethylsilyl)amide, sodium bis(trimethylsilyl)amide, potassium bis(trimethylsilyl)amide, sodium bicarbonate, sodium carbonate, dipotassium hydrogen phosphate, potassium bicarbonate, potassium carbonate, lithium carbonate, caesium carbonate, sodium methoxide, sodium ethoxide, potassium methoxide, potassium ethoxide, sodium tert-butoxide, potassium tert-butoxide, potassium phosphate, sodium hydride, sodium hydroxide, potassium hydroxide, N,N-diisopropylethylamine, triethylamine or 1,8-diazabicycloundec-7-ene, preferably lithium diisopropylamide, lithium bis(trimethylsilyl)amide, sodium bis(trimethylsilyl)amide or potassium bis(trimethylsilyl)amide, more preferably lithium bis(trimethylsilyl)amide, i.e. LiHMDS.

In some embodiments, the solvent used in the reaction of step a is a polar aprotic solvent.

In some embodiments, the polar aprotic solvent used in the reaction of step a is selected from one or more of NN-dimethylformamide, N,N-dimethylacetamide, N-methylpyrrolidone, dichloromethane, 1,2-dichloroethane, ethyl acetate, isopropyl acetate, acetone, n-butanone, methyl tert-butyl ether, dimethylsulfoxide, acetonitrile, diethyl ether, tetrahydrofuran, 2-methyltetrahydrofuran or toluene, preferably tetrahydrofuran or 2-methyltetrahydrofuran, more preferably tetrahydrofuran.

In some embodiments, the molar ratio of formula F-1 : formula E-1 : LiHMDS is 1.0:(1.0-2.0):(0.5-2.0), preferably the molar ratio is 1.0:(1.25-1.75):(1.0-1.5), more preferably the molar ratio is 1.0:(1.3-1.5): (1.1-1.5).

In some embodiments, the temperature of the reaction in step a is -70°C-10°C, preferably -50°C - -10°C, more preferably -40°C - -20°C.

In some embodiments, any of the preparation methods of the present invention comprises a combination of any two or more steps (e.g., three, four, five or six).

In addition, the present invention provides a compound of formula A' or formula A2' in salt form, 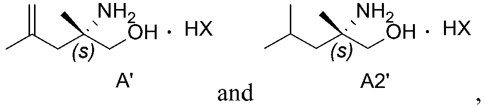 wherein HX is selected from hydrochloric acid, hydrobromic acid, phosphoric acid, trifluoroacetic acid, methanesulfonic acid, benzoic acid, phosphorous acid, formic acid, oxalic acid, malonic acid, sulphuric acid, hydroiodic acid, p-toluenesulfonic acid, maleic acid, fumaric acid, butanedioic acid, L-tartaric acid or L-malic acid.

In an embodiment, the HX is benzoic acid.

Moreover, the present invention provides an intermediate compound of formula A-1:

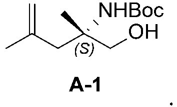

Unless stated to the contrary, the terms used in the description and claims of the present application have the following meanings.

The carbon, hydrogen, oxygen, sulphur and nitrogen or F, Cl, Br and I involved in the groups and compounds of the present invention all comprise their isotopes, and the carbon, hydrogen, oxygen, sulphur or nitrogen involved in the groups and compounds of the present invention is optionally further substituted with one or more of their corresponding isotopes, wherein the isotopes of carbon comprise ¹²C, ¹³C and ¹⁴C; the isotopes of hydrogen comprise protium (H), deuterium (D, also known as heavy hydrogen) and tritium (T, also known as superheavy hydrogen); the isotopes of oxygen comprise ¹⁶O, ¹⁷O and ¹⁸O; the isotopes of sulphur comprise ³²S, ³³S, ³⁴S and ³⁶S; the isotopes of nitrogen comprise ¹⁴N and ¹⁵N; the isotopes of fluorine comprise ¹⁷F and ¹⁹F; the isotopes of chlorine comprise ³⁵Cl and ³⁷Cl; and the isotopes of bromine comprise ⁷⁹Br and ⁸¹Br.

"Halogen" refers to F, Cl, Br or I.

"Halogen-substituted" refers to substitution with F, Cl, Br or I, including but not limited to substitution with 1 to 10 substituents selected from F, Cl, Br or I, substitution with 1 to 6 substituents selected from F, Cl, Br or I, or substitution with 1 to 4 substituents selected from F, Cl, Br or I. "Halogen-substituted" is referred to simply as "halo".

"Alkyl" refers to a substituted or unsubstituted linear or branched saturated aliphatic hydrocarbon group, including but not limited to an alkyl group of 1 to 20 carbon atoms, an alkyl group of 1 to 8 carbon atoms, an alkyl group of 1 to 6 carbon atoms, or an alkyl group of 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched isomers thereof. The definition of the "alkyl" herein is consistent with this definition. Alkyl can be monovalent, divalent, trivalent or tetravalent.

"Cycloalkyl" refers to a substituted or unsubstituted saturated carbocyclic hydrocarbon group, typically having 3 to 10 carbon atoms, and non-limiting examples include cyclopropyl, cyclobutyl, cyclopentyl, cyclohexyl, cycloheptyl, etc. The "cycloalkyl" herein is as defined above. Cycloalkyl can be monovalent, divalent, trivalent or tetravalent.

"Alkoxy" refers to a substituted or unsubstituted -O-alkyl group. Non-limiting examples include methoxy, ethoxy, n-propoxy, isopropoxy, n-butoxy, sec-butoxy, tert-butoxy, n-pentoxy, n-hexyloxy, cyclopropoxy and cyclobutoxy.

"Carbocyclyl" or "carbocycle" refers to a substituted or unsubstituted saturated or unsaturated aromatic ring or non-aromatic ring, wherein the aromatic ring or non-aromatic ring can be a 3- to 8-membered monocyclic ring, 4- to 12-membered bicyclic ring or 10- to 15-membered tricyclic ring system. Carbocyclyl can be connected to an aromatic ring or a non-aromatic ring, wherein the aromatic ring or non-aromatic ring is optionally a monocyclic ring, a bridged ring or a spiro ring. Non-limiting examples include cyclopropane, cyclobutane, cyclopentane, cyclohexane, cycloheptane, 1-cyclopent-1-enyl, 1-cyclopent-2-enyl, 1-cyclopent-3-enyl, cyclohexyl, 1-cyclohex-2-enyl, 1-cyclohex-3-enyl, cyclohexenyl, a benzene ring or a naphthalene ring. "Carbocyclyl" or "carbocycle" can be monovalent, divalent, trivalent or tetravalent.

"Substitution" or "substituted" refers to substitution with 1 or more (including, but not limited to 2, 3, 4 or 5) substituents including, but not limited to H, F, Cl, Br, I, alkyl, cycloalkyl, alkoxy, haloalkyl, mercaptan, hydroxyl, nitro, sulfhydryl, amino, cyano, isocyano, aryl, heteroaryl, heterocyclyl, a bridged ring group, a spiro ring group, a fused ring group, hydroxyalkyl, =O, carbonyl, aldehyde, carboxylic acid, formate, -(CH₂)ₘ-C(=O)-Ra, -O-(CH₂)ₘ-C(=O)-Ra, -(CH₂)ₘ-C(=O)-NRbRc, -(CH₂)_{mS}(=O)ₙRa, -(CH₂)ₘ-alkenyl-Ra, ORd or -(CH₂)ₘ-alkynyl-Ra (wherein m and n are 0, 1 or 2), arylthio, thiocarbonyl, silyl, -NRbRc, etc., wherein Rb and Rc are independently selected from H, hydroxyl, amino, carbonyl, alkyl, alkoxy, cycloalkyl, heterocyclyl, aryl, heteroaryl, sulfonyl or trifluoromethanesulfonyl; alternatively, Rb and Rc may form a five- or six-membered cycloalkyl or heterocyclyl. Ra and Rd are each independently selected from aryl, heteroaryl, alkyl, alkoxy, cycloalkyl, heterocyclyl, carbonyl, an ester group, a bridged ring group, a spiro ring group or a fused ring group.

The salt of a compound refers to a salt obtained by reacting a free acid with an inorganic base or an organic base, or reacting a free base with an inorganic acid or an organic acid.

"Alcohol solvent" refers to a solvent containing hydroxyl in the molecular structure, and non-limiting examples include ethylene glycol, methanol, ethanol, n-propanol, isopropanol, n-butanol, n-pentanol, sec-pentanol, 3-pentanol, isopentanol, tert-pentanol, n-hexanol, cyclohexanol, etc.

"Ether solvent" refers to a solvent containing an ether bond in the molecular structure, and non-limiting examples include tetrahydrofuran, 2-methyltetrahydrofuran, diethyl ether, 1,4-dioxane, methyl tert-butyl ether, ethylene glycol dimethyl ether, diisopropyl ether, ethylbutyl ether, dibutyl ether, dipentyl ether, diethylene glycol dimethyl ether, triethylene glycol dimethyl ether, anisole, etc.

"Aromatic hydrocarbon solvent" refers to a solvent containing an aromatic ring having 0-3 heteroatoms (the heteroatoms are selected from O, S or N) in the molecular structure, and non-limiting examples include benzene, pyridine, toluene, ethylbenzene, xylene, chlorobenzene, o-dichlorobenzene, etc.

"Halogenated alkane solvent" refers to an alkane solvent containing halogen (fluorine, chlorine, bromine or iodine) in the molecular structure, and non-limiting examples include dichloromethane, 1,2-dichloroethane, chloroform, trichloroethane, carbon tetrachloride, pentachlorohexane, 1-chlorobutane, tribromomethane, etc.

"Alkane solvent" refers to a solvent containing only alkane in the molecular structure, and non-limiting examples include n-hexane, n-heptane, n-octane, n-pentane, cyclohexane, cycloheptane, etc.

"Ester solvent" refers to a solvent containing a carboxylic ester in the molecular structure, and non-limiting examples include ethyl acetate, isopropyl acetate, glyceryl triacetate, ethyl acetoacetate, isopentyl acetate, isopropyl acetate, n-butyl acetate, n-propyl acetate, n-pentyl acetate, methyl acetate, sec-butyl acetate, butyl formate, propyl formate, n-pentyl formate, diethyl carbonate, etc.

"Ketone solvent" refers to a solvent containing ketocarbonyl in the molecular structure, and non-limiting examples include acetone, butanone, acetophenone, methyl isobutyl ketone, 2,6-dimethyl-2,5-heptadiene-4-one, 3,5,5-trimethyl-2-cyclohexenone, mesityl oxide, etc.

"Nitrile solvent" refers to a solvent containing cyano in the molecular structure, and non-limiting examples include acetonitrile, propionitrile, butyronitrile, phenylacetonitrile, etc.

"Amide solvent" refers to a solvent containing amide in the molecular structure, and non-limiting examples include N,N-dimethylformamide, N,N-dimethylacetamide, N,N-diethylacetamide, hexamethylphosphoramide, N-methylpyrrolidone, etc.

"Polar aprotic solvent" refers to a solvent that does not contain a hydrogen atom directly connected to an electronegative atom and has no hydrogen bonding ability. Non-limiting examples of polar aprotic solvents include acetone, dimethylsulfoxide, HMF (hydroxymethylfurfural), crown ether, acetonitrile, N,N-dimethylformamide, N,N-diethylformamide, N,N-dimethylacetamide, dimethylsulfoxide, N-methyl-2-pyrrolidone, etc.

"Polar protic solvent" refers to a solvent that has a hydrogen bonding ability (because it contains at least one hydrogen atom directly connected to an electronegative atom (such as O-H or N-H bond)), and non-limiting examples include methanol, water, ethanol, ammonia, acetic acid, etc.

"Optionally" or "alternatively" means that events or circumstances subsequently described may but not necessarily occur, and the description includes the occasion where the events or circumstances occur or do not occur.

During the reaction of the present invention, the reaction process is tracked by HPLC, HNMR or thin layer chromatography so as to judge whether the reaction is completed.

In the present invention, the internal temperature indicates the temperature of the reaction system.

In the present application, "V" or "V/M" refers to the multiple of the volume of the reaction solvent relative to the mass of the starting material fed at 1 eq in the step.

In the present application, the actual amount of starting materials fed or the actual amount of products obtained is determined as follows: if the content is specified, the amount of starting materials or products = the weight of starting materials or products × the content; if the content is not specified, the amount is calculated assuming a 100% content.

Definitions of abbreviations and key terms:

| **Abbreviation code** | **Chinese name** | **Abbreviation code** | **Chinese name** |
|---|---|---|---|
| LiHMDS | Lithium bis(trimethylsilyl)amide | HCl | Hydrochloric acid |
| NaHMDS | Sodium bis(trimethylsilyl)amide | Na₂CO₃ | Sodium carbonate |
| KHMDS | Potassium bis(trimethylsilyl)amide | LDA | Lithium diisopropylamide |
| THF | Tetrahydrofuran | NaBH₄ | Sodium borohydride |
| DCM | Dichloromethane | LiBH₄ | Lithium borohydride |
| MTBE | Methyl tert-butyl ether | I₂ | Iodine |
| THF | Tetrahydrofuran | DCC | N,N'-Dicyclohexylcarbodiimide |
| TEA | Triethylamine | ZnCl₂ | Zinc chloride |
| EtOH | Ethanol | LiAlH₄ | Lithium aluminium hydride |
| MeOH | Methanol | BH₃ | Borane |
| 2-Me-THF | 2-Methyltetrahydrofuran | BH₃_SMe2 | Borane dimethyl sulphide complex |
| n-hep | n-Hexane | CDI | N,N'-Carbonyldiimidazole |
| LiOH | Lithium hydroxide | Pd/C | Palladium on carbon |
| H₂O | Water | H₂ | Hydrogen |
| NaOH | Sodium hydroxide | t-BuOK | Potassium tert-butoxide |
| (Boc)₂O | Di-tert-butyl dicarbonate | CH₃CN | Acetonitrile |

### Technical effects of the present invention

Compared with a chiral resolution preparation method in the prior art, the method for preparing chiral amino alcohol pharmaceutical intermediates of formula A and formula A2 provided by the present invention has the advantages of mild reaction conditions, simple operation, high reaction yield, high product purity, high chiral purity, convenient post-treatment, suitability for industrial production, etc.

### Detailed Description of Embodiments

The present invention is further described in detail below in conjunction with examples which do not limit the present invention. Any equivalent substitutions made according to the disclosure of the present invention fall within the scope of protection of the present invention.

The structures of compounds are determined by nuclear magnetic resonance (NMR) or (and) mass spectrometry (MS). The NMR shift (δ) is given in the unit of 10⁻⁶ (ppm). NMR is determined with (Bruker Avance III 400 and Bruker Avance 300) NMR instrument; the solvents for determination are deuterated dimethylsulfoxide (DMSO-d₆), deuterated chloroform (CDCl₃) and deuterated methanol (CD₃OD); and the internal standard is tetramethylsilane (TMS);
MS is determined with (Agilent 6120B (ESI) and Agilent 6120B (APCI)); and
HPLC is determined with Agilent 1260DAD high pressure liquid chromatograph (Zorbax SC-A18 100 × 4.6 mm, 3.5 µM).

### Example 1: Preparation of compound A and benzoate thereof

The preparation route of compound A is as follows:

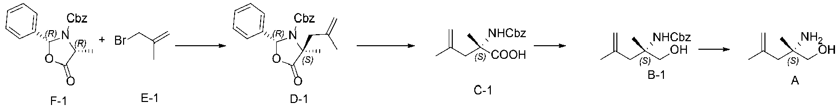

### Step 1: Preparation of D-1 benzyl (2R,4S)-4-methyl-4-(2-methylallyl)-5-oxo-2-phenyloxazolidine-3-carboxylate

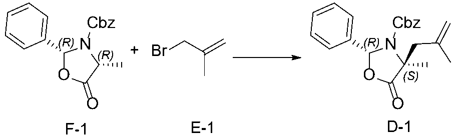

To a dry three-necked round-bottom flask were successively added 0.650 kg of F-1 (1.5 eq.), 7 L of THF (7 V) and 4 L of 1M/L LiHMDS (1.25 eq) at room temperature of 20°C ± 5°C under the protection of high-purity nitrogen. The mixture was cooled to -30°C ± 10°C, and solution of 1.000 kg of E-1 (1.0 eq) in 3 L of THF (3 V) was added dropwise with the temperature controlled (significant exotherm). After the dropwise addition was completed, the internal temperature was maintained at -30°C ± 10°C, and the mixture was stirred for 1.5-2 hours. Samples were taken for IPC-1, confirming completion of the reaction. Post-treatment: 10 L of water (10 V) was added dropwise with the temperature controlled at no more than 25°C. After the dropwise addition was completed, the mixture was stirred for 0.5 h with the temperature maintained at 10°C-20°C, and allowed to stand for phase separation. The aqueous phase was back-extracted once with 5 L of MTBE (5V), and the organic phases were combined. At JT = 45°C ± 5°C and a vacuum degree of 70-200 mbar, the combined organic phase was concentrated under reduced pressure until almost no liquid flowed out, to obtain 1.158 kg of a yellow oil (purity: 98.8%, content: 83.8%, content yield: 83%).
LC-MS: m/z =366.6[M+H]⁺
¹H NMR (400 MHz, DMSO-*d*₆) δ 7.46 - 7.37 (m, 7H), 7.22 - 7.16 (m, 2H), 6.83 - 6.81 (d, 1H), 6.48 - 6.44 (m, 1H), 5.18 - 5.40 (t, 1H), 5.00 - 4.90 (m, 2H), 4.74 - 4.60 (m, 1H),3.21 - 3.17 (d, 1H), 2.46 - 2.41 (m, 1H), 1.77 - 1.64 (m, 6H).

### Step 2: Preparation of C-1

### (S)-2-(((benzyloxy)carbonyl)amino)-2,4-dimethylpent-4-enoic acid

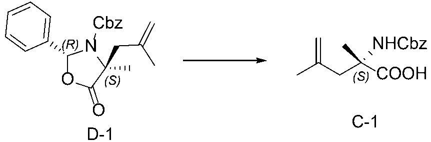

To a dry three-necked round-bottom flask were added 2.5 L of THF (2V) and 1.158 kg of D-1 (1.0 eq, 2.65mol) at room temperature of 20°C. The mixture was cooled to 0°C, and LiOH solution (95.20 g LiOH, 1.5 eq, dissolved in 2.5 L of water) was added dropwise with the temperature controlled at 0°C-10°C. After the dropwise addition was completed, the mixture was warmed to 35°C-40°C, and reacted overnight. Post-treatment at room temperature: the mixture was extracted twice with 5 L of n-hexane (to remove part of benzaldehyde, though incomplete extraction). The aqueous phase was collected, adjusted to pH = 3-4, and extracted again with 5L of EA (5 V). The organic phase was collected, dried over anhydrous sodium sulphate (water in the product would affect the next reaction), and subjected to rotary evaporation, to obtain 882 g of a yellow oil (purity: 90.5%, content: 69.3%, content yield: 83%).
LC-MS: m/z =278.2[M+H]⁺
¹H NMR (400 MHz, DMSO-*d*₆) δ 12.60 (s, 1H), 7.36 - 7.29 (m, 5H), 5.03 - 5.02 (d, 2H), 4.85 - 4.84 (t, 1H), 4.69 (s, 1H), 2.68 - 2.64 (d, 1H), 2.51 - 2.48 (d, 1H), 1.67 (s, 3H), 1.34 (s, 3H).

### Step 3: Preparation of B-1

### benzyl (S)-(1-hydroxy-2,4-dimethylpent-4-en-2-yl)carbamate

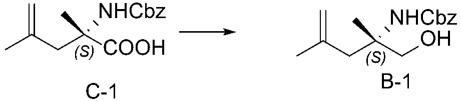

To a 50 L reaction kettle were added 8.7 L of THF (10 V) and 875 g of C-1 (1.0 eq, 2.18 mol) at room temperature. The mixture was cooled to IT = 0°C-5°C, and 446 g of isobutyl chloroformate (1.5 eq, 3.27 mol) was added. Subsequently, 375 g of TEA (1.7 eq, 3.71 mol) was added dropwise with the temperature controlled at 0°C-5°C, and a white solid precipitated. After the dropwise addition was completed, the mixture was stirred for at least 1-1.5 h with the temperature controlled at IT = 0°C-5°C. Samples were taken for in-process control (IPC); upon IPC compliance was confirmed, the mixture was extracted with 10 L of water and 8 L of EA. The organic phase was concentrated to dryness, and the residue was purified by column chromatography (PE:EA 50:1-20:1), to obtain 438.57 g of intermediate as a colourless oil.

To a 10 L four-necked flask were added 438.57 g of intermediate and 4.5 L of THF. The mixture was cooled to 5°C ± 5°C, and 115 g of NaBH₄ (2.5 eq) was added. 500 ml of tap water (1V) was added dropwise over 4-6 hours with the temperature controlled at 5°C ± 5°C, during which bubbling was observed. After the dropwise addition of water was completed, the mixture was stirred for 0.5 h. Samples were taken for in-process control (IPC); upon IPC compliance was confirmed, post-treatment was carried out.

Post-treatment at room temperature: 2.5 L of 10% ammonium chloride (5 V) was added dropwise with the temperature controlled at IT = 10°C ± 10°C. After the dropwise addition was completed, the mixture was warmed to IT = 25°C ± 5°C, stirred for at least 2-3 h, and allowed to stand for phase separation (if a solid residue was present, the mixture was filtered, followed by phase separation). The aqueous phase was extracted with DCM (5V), and the organic phases were combined. The combined organic phase was concentrated until no distillate was observed at JT = 45°C ± 5°C, to obtain 434 g of a colourless oil (purity: 98.5%, content: 92.6%, content yield: 70%).
LC-MS: m/z =264.2[M+H]⁺

### Step 4: Preparation of compound A

### (S)-2-amino-2,4-dimethylpent-4-en-1-ol

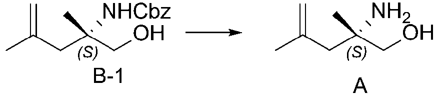

To a 50 L reaction kettle was added 4.5 L of EtOH/H₂O = 10/1 (10V) solution at 25°C ± 5°C. 434 g of A4 (1.0 eq, 1.52 mol) was added with the temperature controlled at 25°C ± 5°C, and 182.4 g of sodium hydroxide (3.0 eq.) was added with the temperature controlled at 30°C ± 10°C. The mixture was warmed to IT = 70°C ± 5°C, and stirred for at least 20 h. Samples were taken for IPC (in case of IPC failure, 1V water and 1.0 eq. NaOH were added, and the mixture was stirred for another 5 h). The mixture was cooled to 20°C ± 5°C, filtered under reduced pressure, and rinsed with ethanol (3 V). The filtrate was concentrated until no distillate was observed at JT = 50°C ± 5°C. To the residual liquid was added water (2V) at 25°C ± 5°C, 10% HCl aqueous solution was added dropwise with the temperature controlled at 25°C ± 5°C, and the mixture was adjusted to pH = 3-5. The mixture was subjected to phase separation based on the pH, and the aqueous phase was retained. The aqueous phase was extracted twice with methyl tert-butyl ether (5V), and the aqueous phase was retained. Sodium hydroxide solution was added to adjust the pH to 11-13, followed by extraction with 2-methyltetrahydrofuran. The organic phase was washed once with saturated aqueous sodium chloride solution, and concentrated to obtain 209 g of HSK41167 as an oil (purity: 89.3%, content: 75%, content yield: 80%).
LC-MS: m/z =130.2[M+H]⁺
¹H NMR (400 MHz, DMSO-*d*₆) δ 4.82 - 4.81 (t, 1H), 4.67 - 4.66 (d, 1H), 3.15 - 3.09 (t, 2H), 2.00 (s, 2H), 1.78 (s, 3H), 0.91 (s, 3H).

### Step 5: Preparation of benzoate of compound A

### (S)-2-amino-2,4-dimethylpent-4-en-1-ol

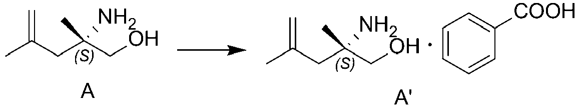

To a 2 L reaction flask was added 0.58 L of DCM (10V) solution at 25°C ± 5°C, followed by addition of 58 g of A (1.0 eq, 0.44 mol) with the temperature controlled at 25°C ± 5°C, and the mixture became a clear solution. 57 g of solid benzoic acid (1.05 eq.) was added portionwise, during which a solid (non-benzoic acid) precipitated from the system. After the addition was completed, the system was stirred for 2 h, filtered under reduced pressure, and rinsed with 50 ml of DCM. The solid was dried in an oven at 50 degrees to obtain 52 g of an off-white product (yield: 45%, purity: 99%).
LC-MS: m/z =130.1 [M+H]⁺
¹H NMR (400 MHz, DMSO-*d*₆) δ 7.91-7.88 (m, 2H), 7.43-7.33 (m, 3H), 4.91-4.90 (m, 1H), 4.79 (m, 1H), 3.40-3.33 (dd, 2H, J=12Hz), 2.31-2.18(d, 1H, J=12Hz), 2.21-2.18(d, 1H, J=12Hz), 1.17 (s, 3H), 1.10 (s, 3H).

### Example 2: Preparation of compound A2 and salt form A2'

### Step 1: Preparation of A2

### (S)-2-amino-2,4-dimethylpentan-1-ol (compound A2)

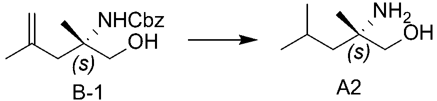

To a 50 ml single-necked flask were added 3.00 g of B-1 (1.0 eq, 0.0114 mol), 30 ml of methanol (10 V) and 0.60 g of 10% Pd/C (20% M) at room temperature. The mixture was purged with H₂ 3 times, warmed to IT = 45°C ± 5°C, and reacted for 12 h. Samples were taken for in-process control (IPC); upon IPC compliance was confirmed, the reaction solution was suction-filtered to remove Pd/C, and methanol was concentrated to dryness. The residue was separated by column chromatography (PE:EA 20:1 - DCM:MeOH 10:1), to obtain 0.82 g of product as a colourless oil (yield: 55%).
LC-MS: m/z =132.1[M+H]⁺
¹H NMR (400 MHz, DMSO-*d*₆) δ 3.07 (s, 2H), 1.78-1.68 (m, 2H), 1.21-1.11 (m, 2H),0.92-0.88 (m, 9H).

### Step 2: Preparation of benzoate A2' of A2

### (S)-2-amino-2,4-dimethylpentan-1-ol benzoic acid (compound A2')

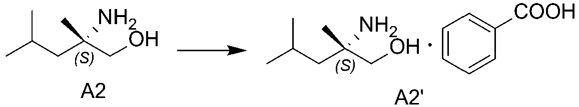

To a 2 L reaction flask was added 0.58 L of DCM (10V) solution at 25°C ± 5°C, followed by addition of 5.0 g of A2 (1.0 eq, 38 mmol) with the temperature controlled at 25°C ± 5°C, and the mixture became a clear solution. 4.89 g of solid benzoic acid (1.05 eq, 40.04 mmol) was added portionwise, during which a solid (non-benzoic acid) precipitated from the system. After the addition was completed, the system was stirred for 2 h, filtered under reduced pressure, and rinsed with 50 ml of DCM. The solid was dried in an oven at 50 degrees to obtain 7.22 g of an off-white product (yield: 75%, purity: 99%).
LC-MS: m/z =132.1[M+H]⁺

### Example 3: Preparation of compound A-1

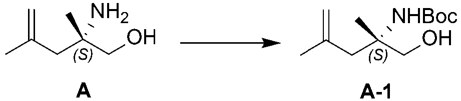

To the aqueous phase were added Na₂CO₃ (1.5 eq.) and methyl tert-butyl ether (5V), (Boc)₂O (2.0 eq.) was added dropwise, and the mixture was stirred for at least 20 h. Samples were taken for in-process control (IPC); upon IPC compliance was confirmed, the mixture was filtered, and subjected to phase separation. The aqueous phase was extracted once with methyl tert-butyl ether (5V), and the organic phases were combined. The combined organic phase was concentrated to about 1.8 w at JT = 45°C ± 5°C. To the residual liquid was added n-heptane (3V), and the mixture was concentrated to about 1.8 w. To the residual liquid was added n-heptane (10V). The mixture was slowly cooled to IT = 5°C ± 5°C, stirred for at least 0.5 h, slowly cooled to IT = -8°C ± 2°C, stirred for at least 2 h, and filtered. The filter cake was rinsed with n-heptane (2V), and air-dried for 12 h to obtain the product (purity as determined by HPLC: 99.7%, yield: 73%).
LCMS m/z =230.10[M+H]⁺
¹H NMR (400 MHz, DMSO-*d*₆) δ 6.08 (s, 1H), 4.81-4.80 (m, 1H), 4.66-4.63 (m, 2H), 3.38-3.30 (m, 2H), 4.49-4.44 (m, 1H), 2.26-2.22(m, 1H), 1.72(s, 3H), 1.40(s, 9H), 1.08 (s, 3H).

### Example 4: Preparation of D-1

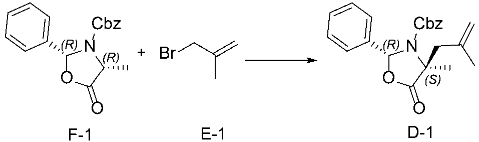

Preparation method: the preparation was carried out with reference to the method of the first step in Example 1. The reaction results under different base systems and conditions are shown in Table 1 below.

**Table 1. Reaction results under different reaction systems and conditions**

| No. | Molar ratio of F1:E1:base | Base | Solvent | Solvent amount | Temperature | D1 | Note |
|---|---|---|---|---|---|---|---|
| 1 | 1.0:1.5:1.25 | LiHMDS | THF | 10 V | 0°C±10°C | 1.11% | |
| 2 | 1.0:1.5:1.25 | LiHMDS | THF | 10 V | -30°C±10°C | 60.04% | |
| 3 | 1.0:1.5:1.25 | LiHMDS | THF | 10 V | -60°C±10°C | 38.3% | |
| 4 | 1.0:1.0:1.25 | LiHMDS | THF | 10 V | -30°C±10°C | 45% | |
| 5 | 1.0:1.5:1.25 | LiHMDS | THF | 10 V | -30°C±10°C | 49.8% | F1 and E1 were added, followed by dropwise addition of LiHMDS. |
| 6 | 1.0:2.0:1.25 | LiHMDS | THF | 10 V | -30°C±10°C | 50.00% | |
| 7 | 1.0:1.0:1.25 | LiHMDS | THF | 10 V | -30°C±10°C | 37.1% | |
| 8 | 1.0:1.0:1.25 | KHMDS | THF | 10V | -30°C±10°C | 1.2% | |
| 10 | 1.0:1.0:1.25 | LDA | THF | 10V | -30°C±10°C | 6.1% | |
| 11 | 1.0:1.0:1.25 | NaHMDS | THF | 10V | -30°C±10°C | 25% | |
| 12 | 1.0:1.0:0.625 | LiHMDS | THF | 10V | -30°C±10°C | 4.8% | |
| 13 | 1.0:1.0:1.25 | LiHMDS | THF | 10V | -30°C±10°C | 63.5% | |
| 14 | 1.0:1.0:1.875 | LiHMDS | THF | 10V | -30°C±10°C | 59.1% | |
| 15 | 1.0:1.5:1.25 | LiHMDS | THF | 10V | -30°C±10°C | 69.7% | F1 and LiHMDS were added, followed by dropwise addition of SM1. |

| | | | | | | | |
|---|---|---|---|---|---|---|---|
| Note: solvent amount: the multiple of the volume of the solvent relative to the mass of F1 fed. | | | | | | | |

Conclusion: the reaction temperature of -30°C ± 10°C was preferable; among the bases tested, LiHMDS gave the optimal reaction effect, followed by NaHMDS, while KHMDS and LDA showed inferior performance; when the molar ratio of F-1 : E-1 : base was 1.0:1.5:1.25, product D-1 was obtained in high yield and the reaction effect was good; the dropwise addition method significantly impacted the reaction, and it was preferable to add LiHMDS first, followed by dropwise addition of F1.

### Example 5: Preparation of C-1

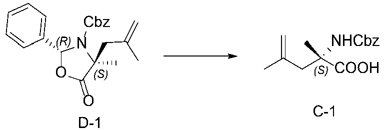

The preparation was carried out with reference to the method of the second step in Example 1. The reaction results under different conditions are shown in Table 2 below.

**Table 2. Reaction conditions screening**

| Reagent | LiOH (1.5 eq.) THF/H₂O (2 V/2 V) |
|---|---|
| Results | D-1: 0%, C-1: 88.9% |

Conclusion: as can be seen from Table 2, under the condition of 1.5 eq. LiOH in THF/H₂O (2 V/2 V), the reaction was carried out at room temperature overnight, and the starting material was completely consumed with an in-process control purity of 88.9%.

### Example 6: Preparation of B-1

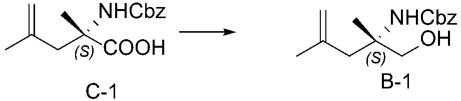

The preparation was carried out with reference to the method of the third step in Example 1. The reaction results under different conditions are shown in Table 3-1 and Table 3-2:

**Table 3-1. Reaction conditions screening**

| No. | Reagent | Solvent | Temperature | C-1 | B-1 |
|---|---|---|---|---|---|
| 1 | NaBH₄ 1.5eq I₂ 0.1eq | THF | rt. | 2.5% | 41.2% |
| 2 | DCC 1.2eq LiBH₄ 1.5eq | THF | rt. | 20.5% | 15.8% |
| 3 | 1) Methyl chloroformate 1.0eq TEA 1.1eq | THF | rt. | 35.0% | 28.9% |
| | 2) NaBH₄ 1.5eq MeOH 1.5eq | | | | |
| 4 | 1) Isobutyl chloroformate 1.0eq TEA 1.1eq | THF | rt. | 28.7% | 34.4% |
| | 2) NaBH₄ 1.5eq MeOH 1.5eq | | | | |
| 5 | NaBH₄ 2.0eq ZnCl₂ 2.0eq | THF | rt. | 47.1% | 0.0% |
| 6 | LiAlH₄ 1.2eq | THF | rt. | 79.0% | 2.3% |
| 7 | BH₃ 3.0eq | THF | rt. | 14.2% | 0% |
| 8 | BH₃ 1.0eq | THF | rt. | 53.3% | 0% |
| 9 | BH₃-SMe₂ 2.0eq | THF | rt. | 4.7% | 0% |
| 10 | NaBH₄ 3.0eq MeOH 3.0eq | THF | rt. | 80.2% | 0% |
| 11 | 1) Isobutyl chloroformate 2.0eq TEA 2.2eq | THF | rt. | 7.7% | 60.2% |
| | 2) NaBH₄ 2.5eq H₂O 2.0V | | | | |
| 12 | CDI 2.0eq NaBH₄ 2.5eq H₂O 1.0V | THF | rt. | 3.0% | 41.6% |

Conclusion: using THF as the solvent for condition screening, isobutyl chloroformate/NaBH₄/water exhibited the best effect.

The preparation was carried out with reference to the method of the third step in Example 1. The reaction results under different conditions are shown in Table 3-2.

**Table 3-2. Reaction results under different reducing systems and conditions**

| No. | Mol ratio of active ester:TEA :NaBH₄: additive | Solvent | Active ester | Additive | C-1 | B-1 |
|---|---|---|---|---|---|---|
| 1 | 1.0:1.1:1.5:1.5 | THF | Methyl chloroformate | MeOH | 35.0% | 28.9% |
| 2 | 1.0:1.1:1.5:1.5 | THF | Isobutyl chloroformate | MeOH | 28.7% | 34.4% |
| 3 | 2.0:2.2:2.5:2.5 | THF | Methyl chloroformate | MeOH | 11.2% | 47.0% |
| 4 | 2.0:2.2:2.5:2.5 | THF | Isobutyl chloroformate | MeOH | 6.8% | 68% |
| 5 | 1.5:1.7:2.5:1V | THF | Isobutyl chloroformate | H₂O | 5.6% | 71.3% |

Conclusion: as can be seen from Table 3-2, the isobutyl chloroformate/NaBH₄/water reduction system exhibited the best effect; the mol ratio of isobutyl chloroformate : TEA : NaBH₄: water selected as 1.5:1.7:2.5:1 V/M provided better results, with 5.6% starting material remaining and 71.3% product yield.

### Example 7: Preparation of compound A

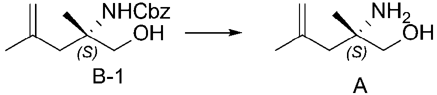

The preparation was carried out with reference to the method of the fourth step in Example 1. The reaction results under different conditions are shown in Table 4-1, Table 4-2 and Table 4-3:

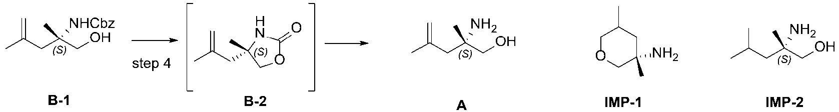

**Table 4-1. Reaction results under different conditions**

| No. | Experimental condition | A | B-1 | Experimental result |
|---|---|---|---|---|
| 1 | TMSI 2.0eq, DCM 10V/M | NA | 0% | IMP-1 was obtained |
| 2 | TMSI 2.0eq, MeCN 10V/M | NA | 0% | IMP-1 was obtained |
| 3 | 33% HBr in AcOH 5V/M | NA | 0% | IMP-1 was obtained |
| 4 | 10% Pd/C 0.3%w H₂ MeOH 10V/M | Small amount | Small amount | Large amount of IMP-2 |
| 5 | 10% Pd/C 0.1%w H₂ MeOH 10V/M | Small amount | Small amount | Large amount of IMP-2 |
| 6 | 10% Pd/C 0.1%w H₂ DCM 10V/M | Small amount | Small amount | Large amount of IMP-2 |
| 7 | 10% Pd/C 0.1%w H₂ EtOH 10V/M | Small amount | Small amount | Large amount of IMP-2 |
| 8 | 10% Pd/C 0.1%w H₂ EA 10V/M | Small amount | Small amount | Large amount of IMP-2 |

| | | | | |
|---|---|---|---|---|
| Note: "IMP-1" denotes impurity 1, and "IMP-2" denotes impurity 2. | | | | |

As can be seen from Table 4-1, removal of the Cbz group with an acid afforded IMP-1. Hydrogenation yielded a large amount of IMP-2.

Under alkaline conditions, fragment B-2 was synthesized, and then hydrolysed to obtain A.

**Table 4-2. Reaction results under different conditions**

| No. | Experimental condition | B-2 | B-1 | Experimental result |
|---|---|---|---|---|
| 1 | 10 V THF as solvent NaOH | 25.9% | 0% | No starting material remaining |
| 2 | 10 V THF as solvent t-BuOK | 22.9% | 0% | No starting material remaining |

As can be seen from Table 4-2, the intermediate B-2 was obtained by base-mediated removal. NaOH was better than t-BuOK.

The hydrolysis reaction using B-2 was investigated:

**Table 4-3. Hydrolysis reaction results under different conditions**

| No. | Experimental condition | B-2 | A | Temperature |
|---|---|---|---|---|
| 1 | 2 V/M EtOH, 10%NaOH 2eq | 17.4% | 6.7% | 70°C±5°C |
| 2 | 10 V/M EtOH, NaOH 3eq | 77.2% | 22.8% | 70°C±5°C |
| 3 | 10 V/M H₂O, NaOH 3eq | 21.2% | 67.0% | 70°C±5°C |
| 4 | 10 V/M EtOH/H₂O=10/1, NaOH 3eq | 0% | 89.3% | 70°C±5°C |

As can be seen from Table 4-3, at IT = 70°C ± 5°C, the conversion to A was achieved under the optimal conditions of EtOH/H₂O = 10/1 as the solvent and 3.0eq. NaOH as the base. Furthermore, B-1 could be directly converted to A under this system.

## Claims

1. A method for preparing an intermediate represented by formula A or a salt thereof, **characterized by** comprising step d1 of subjecting a compound of formula B-1 to a hydrolysis reaction under alkaline conditions to remove the Cbz protecting group to obtain the intermediate of formula A:

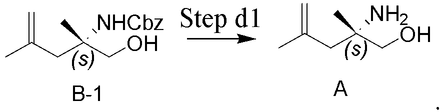

2. The preparation method according to claim 1, **characterized in that** the hydrolysis reagent for the hydrolysis reaction is selected from one or more of sodium hydroxide, potassium carbonate, potassium tert-butoxide, potassium tert-pentoxide, sodium carbonate, caesium carbonate, caesium hydroxide, lithium hydroxide, potassium hydroxide, sodium methoxide or sodium ethoxide, preferably sodium hydroxide or potassium tert-butoxide, more preferably sodium hydroxide.

3. The preparation method according to claim 1 or 2, **characterized in that** the solvent for the hydrolysis reaction is selected from one or more of a polar aprotic solvent or a non-polar solvent.

4. The preparation method according to claim 3, **characterized in that** the solvent for the hydrolysis reaction is selected from one or more of N,N-dimethylformamide, N,N-dimethylacetamide, N-methylpyrrolidone, dichloromethane, 1,2-dichloroethane, ethyl acetate, methyl tert-butyl ether, dimethylsulfoxide, acetonitrile, diethyl ether, tetrahydrofuran, ethanol, methanol or water, preferably at least one of ethanol, tetrahydrofuran or water, more preferably at least one of ethanol or water.

5. The preparation method according to any one of claims 1-4, **characterized in that** the reaction temperature of the hydrolysis reaction is 0°C-90°C, preferably 50°C-80°C, more preferably 70°C ± 5°C.

6. A method for preparing an intermediate represented by formula A2 or a salt thereof, **characterized by** comprising step d2 of subjecting a compound of formula B-1 to a hydrogenation reduction reaction:

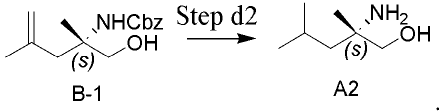

7. The preparation method according to claim 6, **characterized in that** the reducing agent for the reduction reaction is 5% or 10% Pd/C.

8. The preparation method according to claim 7, **characterized in that** the Pd/C is 10% Pd/C, and the amount thereof is 1%-50%, preferably 10%-30%, by mass of the compound of formula B-1.

9. The preparation method according to any one of claims 6-8, **characterized in that** the solvent for the reduction reaction is selected from one or more of N,N-dimethylformamide, N,N-dimethylacetamide, N-methylpyrrolidone, dichloromethane, 1,2-dichloroethane, ethyl acetate, methyl tert-butyl ether, dimethylsulfoxide, acetonitrile, diethyl ether, tetrahydrofuran, ethanol, methanol or water, preferably one or more of ethanol, methanol, ethyl acetate or dichloromethane, more preferably methanol.

10. The preparation method according to any one of claims 6-9, **characterized in that** the reaction temperature of the reduction reaction is 20°C-60°C, preferably 20°C-40°C.

11. A method for preparing an intermediate represented by formula B-1 or a salt thereof, **characterized by** comprising step c of reacting a compound of formula C-1 in the presence of an active agent to obtain a mixed anhydride, followed by reduction to obtain the intermediate of formula B-1:

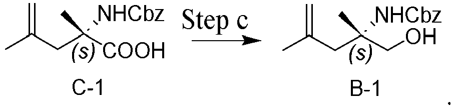

12. The preparation method according to claim 11, **characterized in that** the active agent is selected from methyl chloroformate, ethyl chloroformate, isopropyl chloroformate, butyl chloroformate, isobutyl chloroformate, tert-butyl chloroformate, N,N'-dicyclohexylcarbodiimide or N,N'-carbonyldiimidazole, preferably methyl chloroformate or isobutyl chloroformate, more preferably isobutyl chloroformate.

13. The preparation method according to claim 11 or 12, **characterized in that** the base used during the preparation of the mixed anhydride is selected from potassium carbonate, sodium carbonate, triethylamine, N,N-diisopropylethylamine or diethylamine, preferably triethylamine.

14. The preparation method according to any one of claims 11-13, **characterized in that** the solvent for the reaction in step c is selected from a polar aprotic solvent or a polar protic solvent.

15. The preparation method according to claim 14, **characterized in that** the solvent is selected from one or more of N,N-dimethylformamide, N-methylpyrrolidone, acetonitrile, tetrahydrofuran, 2-methyltetrahydrofuran, acetone, water, methanol or ethanol, preferably tetrahydrofuran.

16. The preparation method according to claim 14, **characterized in that** the solvent is selected from water or a mixture of water and a polar solvent.

17. The preparation method according to claim 16, **characterized in that** the polar solvent is selected from tetrahydrofuran, methanol or ethanol.

18. The preparation method according to any one of claims 11-17, **characterized in that** the reducing agent used in the reaction of step (c) is selected from sodium borohydride or lithium borohydride, preferably sodium borohydride.

19. The preparation method according to any one of claims 11-18, **characterized in that** the additive used in the reaction of step (c) is selected from methanol, ethanol or water, preferably methanol or water, more preferably water.

20. The preparation method according to claim 18 or 19, **characterized in that** the molar ratio of formula B-1 : isobutyl chloroformate : triethylamine : sodium borohydride is 1.0:(1.0-3.0):(1.0-4.0):(1.0-6.0), preferably the molar ratio is 1.0:(1.3-2.5):(1.5-3.0):(2.0-4.0), more preferably the molar ratio is 1.0:(1.3-1.7):(1.5-2.0):(2.5-3.0).

21. The preparation method according to claims 11-20, **characterized in that** the temperature of the reaction in step c is 20°C-40°C.

22. A method for preparing an intermediate represented by formula C-1 or a salt thereof, **characterized by** comprising step b of reacting a compound of formula D-1 in the presence of an alkaline reagent to obtain the intermediate of formula C-1: 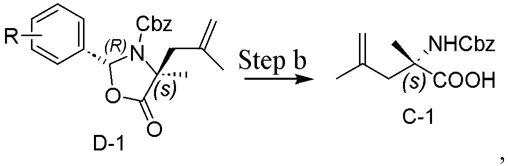 wherein R is selected from H, halogen, C₁₋₄ alkyl, C₁₋₄ alkoxy, C₃₋₆ carbocyclyl, or 4- to 6-membered heterocyclyl, and the alkyl, alkoxy, carbocyclyl or heterocyclyl is optionally substituted with 1 to 4 substituents selected from F, Cl, Br, I, OH, NH₂, C₁₋₄ alkyl, C₁₋₄ alkoxy or C₃₋₆ cycloalkyl.

23. The preparation method according to claim 22, **characterized in that** the R in formula D-1 is H.

24. The preparation method according to claim 22 or 23, **characterized in that** the alkaline reagent is selected from one or more of sodium hydroxide, potassium carbonate, potassium tert-butoxide, potassium tert-pentoxide, sodium carbonate, caesium carbonate, caesium hydroxide, lithium hydroxide, potassium hydroxide, sodium methoxide or sodium ethoxide, preferably lithium hydroxide.

25. The preparation method according to any one of claims 22-24, **characterized in that** the solvent used in the reaction is selected from one or more of amide solvents, alkane solvents, halogenated alkane solvents, alcohol solvents, ketone solvents, ester solvents, ether solvents, nitrile solvents, sulfone solvents or water, preferably one or more of N,Ndimethylformamide, N,N-dimethylacetamide, N-methylpyrrolidone, dichloromethane, 1,2-dichloroethane, ethyl acetate, acetone, methanol, ethanol, isopropanol, n-butanol, trifluoroethanol, ethylene glycol, n-butanone, methyl tert-butyl ether, dimethylsulfoxide, acetonitrile, diethyl ether, tetrahydrofuran and water, more preferably at least one of tetrahydrofuran or water.

26. The preparation method according to claims 22-25, **characterized in that** the temperature of the reaction is -10°C-50°C, preferably 0°C-30°C.

27. A method for preparing an intermediate represented by formula D-1 or a salt thereof, **characterized by** comprising step a of reacting a compound of formula E-1 and a compound of formula F-1 in the presence of an alkaline reagent to obtain the intermediate of formula D-1: 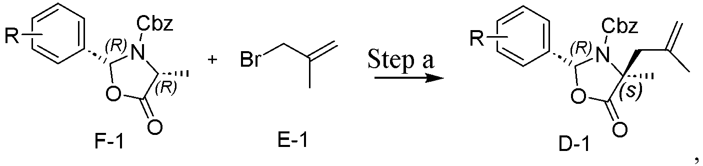 wherein R is selected from H, halogen, C₁₋₄ alkyl, C₁₋₄ alkoxy, C₃₋₆ carbocyclyl, or 4- to 6-membered heterocyclyl, and the alkyl, alkoxy, carbocyclyl or heterocyclyl is optionally substituted with 1 to 4 substituents selected from F, Cl, Br, I, OH, NH₂, C₁₋₄ alkyl, C₁₋₄ alkoxy or C₃₋₆ cycloalkyl.

28. The preparation method according to claim 27, **characterized in that** the R in formulae F-1 and D-1 is H.

29. The preparation method according to claim 27 or 28, **characterized in that** the alkaline reagent is selected from one or more of sodium hydride, lithium diisopropylamide, lithium amide, lithium bis(trimethylsilyl)amide, sodium bis(trimethylsilyl)amide, potassium bis(trimethylsilyl)amide, sodium bicarbonate, sodium carbonate, dipotassium hydrogen phosphate, potassium bicarbonate, potassium carbonate, lithium carbonate, caesium carbonate, sodium methoxide, sodium ethoxide, potassium methoxide, potassium ethoxide, sodium tert-butoxide, potassium tert-butoxide, potassium phosphate, sodium hydride, sodium hydroxide, potassium hydroxide, N,N-diisopropylethylamine, triethylamine or 1,8-diazabicycloundec-7-ene, preferably lithium diisopropylamide, lithium bis(trimethylsilyl)amide, sodium bis(trimethylsilyl)amide or potassium bis(trimethylsilyl)amide, more preferably lithium bis(trimethylsilyl)amide, i.e. LiHMDS.

30. The preparation method according to any one of claims 27-29, **characterized in that** the solvent used in the reaction is a polar aprotic solvent.

31. The preparation method according to claim 30, **characterized in that** the polar aprotic solvent is selected from one or more of N,Ndimethylformamide, N,N-dimethylacetamide, N-methylpyrrolidone, dichloromethane, 1,2-dichloroethane, ethyl acetate, isopropyl acetate, acetone, n-butanone, methyl tert-butyl ether, dimethylsulfoxide, acetonitrile, diethyl ether, tetrahydrofuran, 2-methyltetrahydrofuran or toluene, preferably tetrahydrofuran or 2-methyltetrahydrofuran, more preferably tetrahydrofuran.

32. The preparation method according to any one of claims 29-31, **characterized in that** the molar ratio of formula F-1 : formula E-1 : LiHMDS is 1.0:(1.0-2.0):(0.5-2.0), preferably the molar ratio is 1.0:(1.25-1.75):(1.0-1.5), more preferably the molar ratio is 1.0:(1.3-1.5): (1.1-1.5).

33. The preparation method according to claims 27-32, **characterized in that** the temperature of the reaction is -70°C-10°C, preferably -50°C - -10°C, more preferably -40°C - -20°C.

34. A compound of formula A' or formula A2' in salt form, 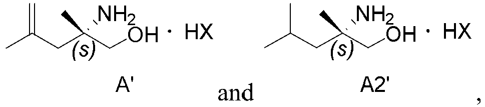 **characterized in that** HX is selected from hydrochloric acid, hydrobromic acid, phosphoric acid, trifluoroacetic acid, methanesulfonic acid, benzoic acid, phosphorous acid, formic acid, oxalic acid, malonic acid, sulphuric acid, hydroiodic acid, p-toluenesulfonic acid, maleic acid, fumaric acid, butanedioic acid, L-tartaric acid or L-malic acid, preferably benzoic acid.

35. An intermediate compound of formula A-1:
